(19) Europäisches Patentamt
European Patent Office
Office européen des brevets

(11)  EP 4 667 048 A1

(12)  EUROPEAN PATENT APPLICATION
published in accordance with Art. 153(4) EPC

(43) Date of publication:
24.12.2025  Bulletin 2025/52

(51) International Patent Classification (IPC):
A61N 1/40 $^{(2006.01)}$          A61N 1/32 $^{(2006.01)}$
A61N 1/36 $^{(2006.01)}$          A61N 1/06 $^{(2006.01)}$
A61B 5/0531 $^{(2021.01)}$        A61B 5/01 $^{(2006.01)}$
A61B 5/00 $^{(2006.01)}$

(21) Application number: 24757066.6

(22) Date of filing: 26.01.2024

(52) Cooperative Patent Classification (CPC):
A61B 5/00; A61B 5/01; A61B 5/0531; A61N 1/06;
A61N 1/32; A61N 1/36; A61N 1/40

(86) International application number:
PCT/KR2024/001269

(87) International publication number:
WO 2024/172336 (22.08.2024 Gazette 2024/34)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority:  13.02.2023  KR 20230018641

(71) Applicant: VIOL CO., LTD.
Seongnam-si, Gyeonggi-do 13510 (KR)

(72) Inventors:
• KIM, Woosung
  Bucheon-si Gyeonggi-do 14782 (KR)
• PARK, Yong Seok
  Seoul 06326 (KR)

(74) Representative: Maiwald GmbH
Elisenhof
Elisenstraße 3
80335 München (DE)

(54)  **SKIN TREATMENT DEVICE AND CONTROL METHOD THEREFOR**

(57)  A skin treatment device according to the present disclosure includes: a radio frequency (RF) generator for generating RF energy; an electrode for transmitting the RF energy to a skin tissue of a user; a measurement unit for measuring a forward RF voltage caused by the RF energy and a reverse RF voltage caused by the RF energy reflected to the RF generator; and a controller for calculating skin impedance of the user and RF output power efficiency based on the measured forward RF voltage and reverse RF voltage, and controlling outputs of the RF generator based on the calculated skin impedance and RF output power efficiency.

FIG. 1

EP 4 667 048 A1

**Description**

**[Technical Field]**

The present disclosure relates to a skin treatment device and a control method thereof. In particular, the present disclosure relates to a skin treatment device for measuring output power efficiency and impedance according to skin impedance in real-time, and a control method thereof, regarding RF treatment devices.

**[Background Art]**

Recently, RF treatment, which delivers RF energy by inserting a needle-shaped electrode into skin tissues, has been widely used for the treatment of skin lesions such as wrinkle removal, scar removal, and acne treatment.

The RF treatment method uses the principle that when a RF current is supplied to the tissues through an electrode, electrical energy flowing through the tissues is converted into heat energy and the energy is transferred to the tissue. At this time, when transmitting RF energy with the same output, the transmitted energy may differ depending on impedance characteristics of the skin (target tissues). Due to these characteristics, when the treatment is performed under the same condition, problems may occur in which sufficient energy is not delivered, preventing optimal treatments, or excessive energy is applied, causing damages to tissues.

Accordingly, RF treatment devices for delivering RF energy suitable for tissues by considering the impedance characteristics of the tissues are disclosed in Prior Art 1 (Patent Laid-Open Publication No. 10-2018-0111202) and Prior Art 2 (Patent Laid-Open Publication No. 10-2022-0129344).

However, in the case of Prior art 1 and Prior art 2, a method for calculating the impedance of tissues by measuring the current, voltage, and power applied to the electrode when RF energy is applied is proposed. That is, forward RF current and voltage by which RF energy is transmitted to the skin tissues is measured so reverse RF current and voltage reflected from the skin tissues are not considered. Therefore, there is a problem in that the impedance of skin tissues for the RF energy delivered to the skin tissues may not be accurately calculated. There is a limitation in that it may not measure the impedance of skin tissues in real time by emitting a predetermined level of RF energy for a predetermined period of time and measuring the flowing current.

**[Disclosure]**

**[Technical Problem]**

The present disclosure attempts to provide a skin treatment device for measuring RF output power efficiency and skin impedances in real-time in a RF treatment device, and a control method thereof.

The present disclosure attempts to provide a skin treatment device for accurately measuring RF output power efficiency and skin impedances by considering forward RF energy output by a RF treatment device and reverse RF energy reflected from skin tissues, and a control method thereof.

The present disclosure attempts to provide a skin treatment device for controlling to quantitatively output RF energy according to a user skin characteristic by measuring RF output power efficiency and skin impedances in real-time, and a control method thereof.

**[Technical Solution]**

An embodiment of the present disclosure provides a skin treatment device including: a radio frequency (RF) generator for generating RF energy; an electrode for transmitting the RF energy to a skin tissue of a user; a measurement unit for measuring a forward RF voltage caused by the RF energy and a reverse RF voltage caused by the RF energy reflected to the RF generator; and a controller for calculating skin impedance of the user and RF output power efficiency based on the measured forward RF voltage and reverse RF voltage, and controlling outputs of the RF generator based on the calculated skin impedance and RF output power efficiency.

The controller may include a data processor for calculating the skin impedance of the user and the RF output power efficiency based on the measured forward RF voltage and reverse RF voltage, and a RF controller for controlling output parameters of the RF energy based on the calculated skin impedance and RF output power efficiency.

The measurement unit may be connected to a RF transmission line for transmitting RF energy output by the RF generator to the electrode, and may measure a forward RF voltage caused by RF energy output by the RF generator and a reverse RF voltage caused by the RF energy reflected from a load including the skin tissue of the user.

The measurement unit may include a directional power coupler module.

The measurement unit may measure the forward RF voltage and the reverse RF voltage as voltage root mean squares, respectively.

The data processor may include first and second analog-to-digital converters for converting the forward RF voltage and the reverse RF voltage measured by the measurement unit into digital signals, and a digital processing module for calculating skin impedance and RF output power efficiency based on the converted forward RF voltage and the reverse RF voltage.

The data processor may calibrate measurement errors when a load of the measurement unit is changed to a first load, a second load, and a third load operable as Open, Short, and Load, may calculate the skin impedance (Z) as

$$Z = Z_0 \frac{V_F - V_R}{V_F + V_R}$$ based on a forward voltage ($V_F$) and a reverse voltage ($V_R$) measured at an input point of the measurement unit, may calculate RF output power efficiency ($P_R / P_F$) as $\dfrac{V_R^2}{V_F^2}$ based on the forward voltage ($V_F$)

and the reverse voltage ($V_R$), here, $Z_0$ may represent a load value that corresponds to a rated load and may be given as 50Ω, and $P_F$ and $P_R$ may represent forward RF electric power and reverse RF electric power.

The data processor may calculate temperature variation inside the skin tissue of the user for respective output levels of the RF energy based on the calculated skin impedance.

The controller may control the output parameter of the RF energy based on at least one of the calculated skin impedance, the RF output power efficiency, and the temperature variation inside the skin tissue, and the output parameter of the RF energy may be at least one of a voltage size and an output time of the RF energy.

The skin treatment device may further include a display unit, wherein at least one of the calculated skin impedance of the user and the RF output power efficiency may be output to the display unit.

Another embodiment of the present disclosure provides a method for controlling a skin treatment device including: generating RF energy; measuring a forward RF voltage caused by the RF energy and a reverse RF voltage caused by the RF energy reflected to a RF generator; calculating skin impedance of a user and RF output power efficiency based on the measured forward RF voltage and the reverse RF voltage; and controlling an output of the RF generator based on the calculated skin impedance and the RF output power efficiency.

**[Advantageous Effects]**

According to the embodiments of the present disclosure, the following effects may be achieved.

According to at least one of the embodiments of the present disclosure, the skin treatment device for measuring the RF output power efficiency and skin impedances in real-time in the RF treatment device, and the control method thereof may be provided.

According to at least one of the embodiments of the present disclosure, the skin treatment device for accurately measuring the RF output power efficiency and skin impedances by considering the forward RF energy output by the RF treatment device and the reverse RF energy reflected from the skin tissue, and the control method thereof may be provided.

According to at least one of the embodiments of the present disclosure, the skin treatment device for measuring the RF output power efficiency and skin impedances in real-time and controlling to quantitively output the RF energy according to the user skin characteristic, and the control method thereof may be provided.

**[Description of the Drawings]**

FIG. 1 shows a block diagram on a configuration of a skin treatment device according to an embodiment.
FIG. 2 shows a block diagram on a detailed configuration of a skin treatment device according to an embodiment.
FIG. 3 shows a circuit diagram on an example of a directional power coupler according to an embodiment.
FIG. 4 shows a graph on changes of skin impedance and RF output power efficiency in real-time according to an embodiment.
FIG. 5 shows an operational flowchart on a method for controlling a skin treatment device according to an embodiment.

**[Mode for Invention]**

Embodiments disclosed in the present specification will be described in detail with reference to the accompanying drawings. In the present specification, the same or similar components will be denoted by the same or similar reference numerals, and an overlapped description thereof will be omitted. The terms "module" and "unit" for components used in the following description are used only in order to make the specification easier, and hence, these terms do not have meanings or roles that distinguish them from each other by themselves.

In describing embodiments of the present specification, when it is determined that a detailed description of the well-known art associated with the present invention may obscure the gist of the present invention, it will be omitted.

The accompanying drawings are provided only in order to allow embodiments disclosed in the present specification to be easily understood and are not to be interpreted as limiting the spirit disclosed in the present specification, and it is to be understood that the present invention includes all modifications, equivalents, and substitutions without departing from the scope and spirit of the present invention.

Terms including ordinal numbers such as first, second, and the like, will be used only to describe various components, and are not interpreted as limiting these components. The terms are only used to differentiate one component from others.

It is to be understood that when one component is referred to as being "connected" or "coupled" to another component, it may be connected or coupled directly to another component or be connected or coupled to another component with the other component intervening therebetween. On the other hand, it is to be understood that when one component is referred to as being "connected or coupled directly" to another component, it may be connected to or coupled to another component without the other component intervening therebetween.

A skin treatment device according to an embodiment and a control method thereof will now be described in detail with reference to accompanying drawings.

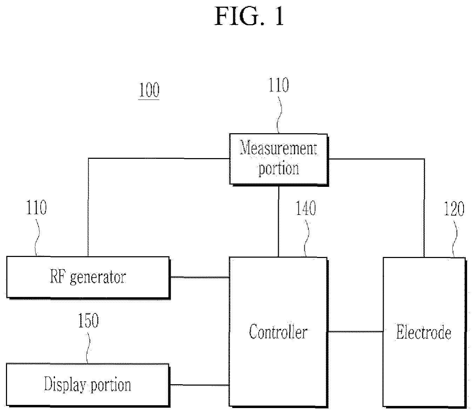

FIG. 1 shows a block diagram on a configuration of a skin treatment device 100 according to an embodiment.

Referring to FIG. 1, the skin treatment device 100 may include a RF generator 110, an electrode 120, a measurement unit 130, and a controller 140.

The components illustrated in FIG. 1 are not essential for implementing the skin treatment device 100, and thus the skin treatment device 100 described in the present specification may have more or fewer components than the components listed above.

The RF generator 110 may receive a power voltage from a power supply (not shown) and may generate RF energy. The RF energy output by the RF generator 110 may have changeable voltage sizes and output times.

The electrode 120 may transmit the RF energy generated by the RF generator 110 to skin tissues of a user. The electrode 120 may have at least one or more needle shape, and may transmit the RF energy to points inside the skin tissues of the user through an end of the needle.

The measurement unit 130 may be connected to a RF transmission line between the RF generator 110 and the electrode 120 and may measure a forward RF voltage caused by the RF energy output by the RF generator 110 and a reverse RF voltage caused by the RF energy reflected to the RF generator 110. According to an embodiment, the measurement unit connected to the RF transmission line may include a directional power coupler module.

The controller 140 may calculate skin impedance and RF output power efficiency of the user in real-time by using the forward RF voltage and the reverse RF voltage measured by the measurement unit 130, and may control outputs of the RF generator 110. That is, the controller 140 may control output parameters including a voltage size and an output time of the RF energy output by the RF generator 110 based on the calculated skin impedance and RF output power efficiency.

The skin treatment device 100 may further include a display unit 150 for outputting information processed by a device.

The display unit 150 may further display: executing screen information on an application program driven by the skin treatment device 100, a user interface (UI) following the executing screen information, and graphic user interface (GUI) information. For example, the display unit 150 may output measurement information including skin impedance and RF output power efficiency and treatment information including output parameters of RF energy in real-time.

The display unit 150 may have a touch screen for receiving instructions of the user, and outputting measurement information and treatment information in real-time, but is not limited thereto.

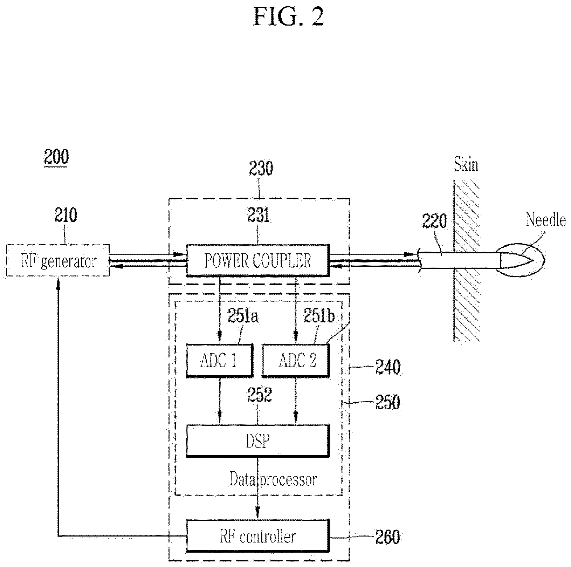

FIG. 2 shows a block diagram on a detailed configuration of a skin treatment device according to an embodiment.

Regarding the RF treatment device, a portion of the RF energy output by the RF generator 210 may be reflected by the load including skin tissues and may be transmitted to the RF generator. Hence, the skin treatment device according to an embodiment proposes a method for considering reverse RF energy provided to the RF generator and accurately calculating the RF energy and skin impedance transmitted to the actual skin.

Referring to FIG. 2, a measurement unit 230 of the skin treatment device may be connected to the RF transmission line between the RF generator 210 and the electrode 220, and may measure a forward RF voltage ($V_F$) caused by forward RF energy output by the RF generator 210 and a reverse RF voltage ($V_R$) caused by reverse RF energy reflected to the RF generator 110. According to an embodiment, the measurement unit 230 may include a directional power coupler module 231 to measure the forward RF voltage ($V_F$) and the reverse RF voltage ($V_R$).

The directional power coupler may be used by a circuit for measuring impedance mismatch of the RF transmission line. The directional power coupler has a forward transmission path and a coupling transmission path. By the above-noted configuration, the voltage (forward voltage) caused by the forward RF energy from a RF source to the load and the voltage (reverse voltage) caused by reverse RF energy reflected to the source from the load may be measured.

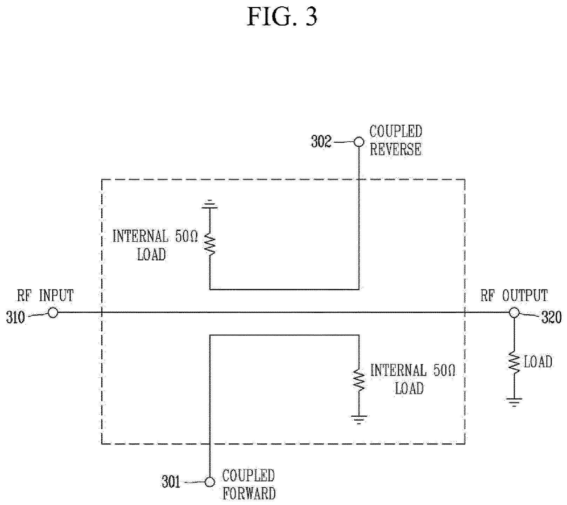

FIG. 3 shows an example of a directional power coupler installed in a skin treatment device according to an embodiment.

Referring to FIG. 3, the forward RF voltage ($V_F$) caused by the RF energy output by the RF generator and input to the RF Input port 310 may be measured by a coupled forward port 301. The reverse RF energy reflected from the load including skin tissues and input to the RF output port 320 may be measured as the reverse RF voltage ($V_R$) at the coupled reverse port 302. The internal load shown in FIG. 3 represents internal resistance for measuring the forward RF voltage ($V_F$) and the reverse RF voltage ($V_R$), and it may be given as 50$\Omega$ in an embodiment of the present disclosure, but is not limited thereto. The load shown in FIG. 3 may represent a load including skin tissues.

Referring to FIG. 2, a controller 240 of the skin treatment device may include a data processor 250 and a RF controller 260.

The data processor 250 may include a first analog-to-digital converter (ADC1) 251a and a second analog-to-digital converter (ADC2) 251b to express the forward RF voltage ($V_F$) and the reverse RF voltage ($V_R$) as digital values.

The forward RF voltage ($V_F$) and the reverse RF voltage ($V_R$) measured by the directional power coupler module 231 of the measurement unit 230 may be alternating current (AC) signals. The sizes and the directions of the AC signals change periodically with respect to time so instantaneous values, maximum values, average values, P-P(Peak-to-Peak) values, or root mean squares may be used to express the sizes of the AC signals as numbers.

According to an embodiment, alternating current (AC) voltages caused by the forward RF energy and the reverse RF energy may be measured as the root mean square (RMS).

The root mean square is a method for measuring the AC voltage as a DC voltage, or a digital value, based on the principle that the electrical energy consumed in the resistance of a DC circuit is equal to the electrical energy consumed in an AC circuit of the same resistance. The root mean square ($V_{rms}$) of the AC voltage may be calculated as expressed in Equation 1 by using a maximum value ($V_m$) of the AC voltage.

(Equation 1)

$$V_{rms} = \frac{1}{\sqrt{2}} V_m = 0.707 V_m [V]$$

Referring to FIG. 2, the data processor 250 may further include a digital signal processing (DSP) module 252 for calculating the skin impedance and RF energy efficiency using the forward RF voltage ($V_F$) and reverse RF voltage ($V_R$) measured as root mean squares.

The calculation of skin impedance according to the present specification may be based on a short open load (SOL) calibrating algorithm used by a vector network analyzer (VNA).

In detail, a method for calculating skin impedance using the SOL calibrating algorithm will be as follows. Referring to FIG. 2 and FIG. 3, the measurement unit 230 may be realized with the vector network analyzer. The measurement unit 230 and the controller 240 may be realized with the vector network analyzer.

The forward voltage measured by the coupled forward port 301 of FIG. 3 may be defined as X, the reverse voltage measured by the coupled reverse port 302 may be defined as Y, and impedance of the skin that corresponds to the load may be defined as Z.

Before measuring the skin impedance, the load may be changed to a first load, a second load, and a third load operable as Open, Short, and Load in a corresponding frequency bandwidth, and measured errors of the vector network analyzer may be calibrated.

In connection with this, the forward voltage X1 and the reverse voltage Y1 in an open circuit of which the load value is set as Open may be measured, and the forward voltage X2 and the reverse voltage Y2 in a short-circuit of which the load value is set as Short may be measured. The forward voltage X3 and the reverse voltage Y3 may be measured in a circuit of which the load value is set as a rated load of 50$\Omega$.

The DSP module 252 may measure the forward voltage ($V_F$) and the reverse voltage ($V_R$) while the load including the skin tissues of the user, that is, the forward voltage ($V_F$) and the reverse voltage ($V_R$) is connected in the circuit of FIG. 3.

In connection with this, the vector network analyzer may be calibrated using the forward voltages X1 to X3 and the reverse voltages Y1 to Y3 measured by the above-noted method, and the skin impedance Z may be calculated.

Referring to FIG. 3, the forward voltage ($V_F$) and the reverse voltage ($V_R$) are measured at an input point of the vector network analyzer. A load impedance corresponds to the skin impedance Z at a load point connected to an output point of the vector network analyzer. Therefore, assuming that the vector network analyzer is a transmission line, the skin impedance Z at an output point of the transmission line may be calculated as expressed in Equation 2, using the forward voltage ($V_F$) and the reverse voltage ($V_R$) at an input point of the transmission line.

(Equation 2)

$$Z = Z_0 \frac{V_F - V_R}{V_F + V_R}$$

In connection with this, $Z_0$ is a load value that corresponds to the rated load, and may be set to be 50Ω. $V_F$ corresponds to the forward voltage measured at the coupled forward port 301, and $V_R$ corresponds to the reverse voltage measured at the coupled reverse port 302.

Forward RF electric power ($P_F$) caused by the forward RF energy and reverse RF electric power ($P_R$) caused by the reverse RF energy may be calculated, using the measured forward voltage ($V_F$), the reverse voltage ($V_R$), and the skin impedance Z calculated by Equation 2, according to Equation 3.

(Equation 3)

$$P_F = \frac{V_F^2}{Z}$$

$$P_R = \frac{V_R^2}{Z}$$

The DSP module 252 may calculate RF output power efficiency using the forward RF electric power ($P_F$) and the reverse RF electric power ($P_R$) according to Equation 4.

(Equation 4)

$$RF\ Output\ Power\ Efficiency = \frac{P_R}{P_F}$$

Referring to FIG. 2 and FIG. 3, the data processor 250 may calculate the skin impedance (Z) and RF output power efficiency ($P_R/P_F$) based on Equations 2 to 4. In connection with this, the data processor 250 may calibrate the measurement errors when the load of the measurement unit 230 is changed to the first load, the second load, and the third load operable as Open, Short, and Load. The data processor 250 may calculate the skin impedance (Z) with $Z = Z_0 \frac{V_F - V_R}{V_F + V_R}$ based on the measured forward voltage ($V_F$) and the reverse voltage ($V_R$) measured at the input point of the measurement unit 230. The data processor 250 may calculate the RF output power efficiency ($P_R/P_F$) with $\frac{V_R^2}{V_F^2}$ based on the forward voltage ($V_F$) and the reverse voltage ($V_R$). Here, $Z_0$ is a load value that corresponds to the rated load and is 50Ω, and $P_F$ and $P_R$ are forward RF electric power and reverse RF electric power.

Hence, the method for calculating skin impedance according to the present disclosure has a merit in allowing fast and accurate calculation as expressed in Equation 2 based on the forward voltage ($V_F$) and the reverse voltage ($V_R$) at the input point of the measurement unit with no complicated operation process. Particularly, the measured error of the measurement system may be calibrated through the first to third loads of Open, Short, and Load having a standard configuration, and the skin impedance may be calculated fast and accurately.

Regardless of a length error from a needle going into the skin to the input point of the measurement unit, the skin impedance may be calculated based on the forward voltage ($V_F$) and the reverse voltage ($V_R$) at the input point of the measurement unit.

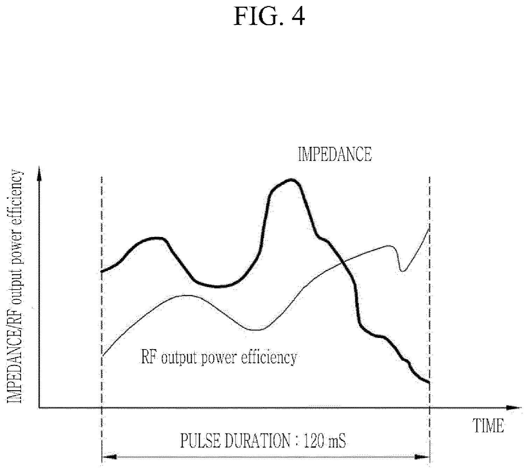

FIG. 4 shows a graph on changes of skin impedance and RF output power efficiency with respect to time according to an embodiment.

The skin treatment device may measure the changes of skin impedance and RF output power efficiency with respect to time for respective RF energy output times, that is, pulse duration (e.g., 120Ms). The changes of the measured skin impedance and RF output power efficiency may be in real-time to the display unit of the skin treatment device.

BY this, the user of the RF treatment device may monitor skin impedance characteristics that are different for respective users in real-time and may control output of RF energy. That is, the RF energy may be quantitively irradiated according to the user skin impedance by adjusting the output time of RF energy or the voltage size of RF energy.

The skin impedance and RF output power efficiency calculated in real-time by the above-noted method may be stored in a memory (not shown) further included in the skin treatment device in real-time. The skin impedance characteristics for respective users may be obtained based on the skin impedance and RF output power efficiency for respective users stored in the memory, which may be helpful in quantitatively irradiating RF energy according to the user skin impedance characteristics.

Although not shown in the drawing, changes of the temperature inside the skin of the user may be monitored in real-time based on the skin impedance and RF output power efficiency calculated by the above-noted method.

Referring to FIG. 2, the RF controller 260 of the skin treatment device according to an embodiment may control the output of the RF energy output by the RF generator 210 based on the calculated skin impedance and RF output power efficiency. That is, the RF controller 260 may automatically control output parameters of the RF energy output by the RF generator 210 according to a predetermined condition according to the skin impedance and RF output power efficiency calculated differently for the respective users.

For example, when the calculated RF output power efficiency is less than a predetermined threshold value, the output time of the RF energy or the voltage size of the RF energy may be increased by a predetermined value. When the calculated RF output power efficiency is greater than a predetermined threshold value, the output time of the RF energy or the voltage size of the RF energy may be reduced by a predetermined value. When variation of the RF output power efficiency is substantially increased or reduced, the output of the RF energy may be intercepted, but is not limited thereto.

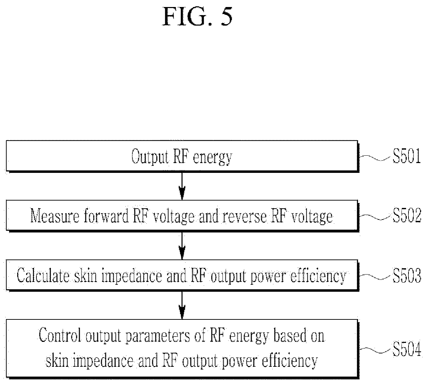

A basic operation of the skin treatment device will now be described with reference to FIG. 5.

The skin treatment device outputs RF energy through the RF generator (S501).

The RF energy output by the RF generator may be adjusted so that output parameters such as the voltage size and the output time of RF energy may be changed. The RF energy output by the RF generator may be transmitted to the skin tissues of the user through the electrode connected to the RF transmission line.

The skin treatment device may respectively measure the forward RF voltage caused by the RF energy output by the RF generator and the reverse RF voltage caused by the RF energy reflected to the RF generator (S502).

The measurement unit of the skin treatment device may be connected to the RF transmission line between the RF generator and the electrode and may measure the forward RF voltage caused by the forward RF energy output by the RF generator. The measurement unit may measure the reverse RF voltage caused by the RF energy reflected from the load including the skin tissue of the user.

The measurement unit according to an embodiment may include a directional power coupler module to measure the forward RF voltage and the reverse RF voltage. The directional power coupler represents a module used by a circuit for measuring impedance mismatch of the RF transmission line and has a forward transmission path and a coupling transmission path. By the above-noted configuration, the voltage (or forward voltage) caused by the forward RF energy from the RF source to a load and the voltage (or reverse voltage) caused by the reverse RF energy reflected to a source from the load may be measured.

The skin treatment device calculates skin impedance and RF output power efficiency based on the measured forward RF voltage and reverse RF voltage (S503).

The forward RF voltage and the reverse RF voltage measured by the directional power coupler of the measurement unit may be alternating current (AC) signals. Hence, the forward RF voltage and the reverse RF voltage measured as the AC signals may be sampled into digital signals by the first and second analog-to-digital converter modules and they may be calculated as root mean squares. The root mean square is a value obtained by measuring the AC voltage as a DC voltage, or a digital value, based on the principle that the electrical energy consumed in the resistance of a DC circuit is equal to the electrical energy consumed in an AC circuit of the same resistance. The forward RF voltage and the reverse RF voltage measured as the root mean squares may be calculated as skin impedance and RF output power efficiency by a digital signal processing (DSP) module of the controller.

The DSP module may calculate skin impedance based on a Short Open Load (SOL) calibrating algorithm used by a vector network analysis (VNA).

In detail, a method for calculating skin impedance using the SOL calibrating algorithm will now be described.

Before measuring the skin impedance, the load may be changed to a first load, a second load, and a third load operable as Open, Short, and Load in a corresponding frequency bandwidth, and measured errors of the vector network analyzer may be calibrated.

In connection with this, the forward voltage X1 and the reverse voltage Y1 in an open circuit of which the load value is set as Open may be measured, and the forward voltage X2 and the reverse voltage Y2 in a short-circuit of which the load value is set as Short may be measured. The forward voltage X3 and the reverse voltage Y3 may be measured in a circuit of which the load value is set as a rated load of 50Ω.

The forward voltage ($V_F$) and the reverse voltage ($V_R$) may be measured while the forward voltage ($V_F$) and the reverse voltage ($V_R$), that is, the load including the skin tissue of the user are connected. In connection with this, the vector network analyzer may be calibrated using the forward voltages X1 to X3 and the reverse voltages Y1 to Y3 measured by the above-noted method, and the skin impedance Z may be calculated.

The DSP module may respectively calculate the forward RF electric power ($P_F$) caused by forward RF energy and the reverse RF electric power ($P_R$) caused by reverse RF energy by using the measured forward voltage ($V_F$) and the reverse voltage ($V_R$), and the calculated skin impedance Z. The DSP module may calculate the RF output power efficiency

$$( P_R/P_F )\quad \frac{V_R{}^2}{V_F{}^2}$$ by using the forward RF electric power ($P_F$) and the reverse RF electric power ($P_R$).

The skin treatment device may control output parameters of RF energy based on the calculated skin impedance and the RF output power efficiency (S504). In detail, the controller may adjust the output parameters including the voltage size and output time of the RF energy based on the calculated skin impedance and RF output power efficiency.

The above-described present disclosure may be realized as a computer readable code on a medium on which a program is recorded. The computer readable medium includes any kinds of recording devices in which data readable by a computer system are stored. The computer readable medium exemplifies a hard disk drive (HDD), a solid state disk (SSD), a silicon disk drive (SDD), a ROM, a RAM, a CD-ROM, a magnetic tape, a floppy disk, and an optical data storage device. The computer may also include a controller of the skin treatment device.

Therefore, the detailed description is not restrictively analyzed at all aspects but considered as an example. The scope of the present disclosure needs to be determined by a reasonable analysis of appended claims and all changes within an equivalent scope of are included in the scope of the present disclosure.

**Claims**

1. A skin treatment device comprising:

   a radio frequency (RF) generator for generating RF energy;
   an electrode for transmitting the RF energy to a skin tissue of a user;
   a measurement unit for measuring a forward RF voltage caused by the RF energy and a reverse RF voltage caused by the RF energy reflected to the RF generator; and
   a controller for calculating skin impedance of the user and RF output power efficiency based on the measured forward RF voltage and reverse RF voltage, and controlling outputs of the RF generator based on the calculated skin impedance and RF output power efficiency.

2. The skin treatment device of claim 1, wherein

   the controller includes
   a data processor for calculating the skin impedance of the user and the RF output power efficiency based on the measured forward RF voltage and reverse RF voltage, and
   a RF controller for controlling output parameters of the RF energy based on the calculated skin impedance and RF output power efficiency.

3. The skin treatment device of claim 1, wherein
   the measurement unit is connected to a RF transmission line for transmitting RF energy output by the RF generator to the electrode, and measures a forward RF voltage caused by RF energy output by the RF generator and a reverse RF voltage caused by the RF energy reflected from a load including the skin tissue of the user.

4. The skin treatment device of claim 3, wherein
the measurement unit includes a directional power coupler module.

5. The skin treatment device of claim 3, wherein
the measurement unit measures the forward RF voltage and the reverse RF voltage as voltage root mean squares, respectively.

6. The skin treatment device of claim 2, wherein

the data processor includes
first and second analog-to-digital converters for converting the forward RF voltage and the reverse RF voltage measured by the measurement unit into digital signals, and
a digital processing module for calculating skin impedance and RF output power efficiency based on the converted forward RF voltage and the reverse RF voltage.

7. The skin treatment device of claim 6, wherein

the data processor calibrates measurement errors when a load of the measurement unit is changed to a first load, a second load, and a third load operable as Open, Short, and Load, calculates the skin impedance (Z) as

$$Z = Z_0 \frac{V_F - V_R}{V_F + V_R}$$ based on a forward voltage ($V_F$) and a reverse voltage ($V_R$) measured at an input point of the measurement unit, calculates RF output power efficiency ($P_R / P_F$) as $\frac{V_R{}^2}{V_F{}^2}$ based on the forward voltage

($V_F$) and the reverse voltage ($V_R$),
here, $Z_0$ represents a load value that corresponds to a rated load and is given as 50Ω, and $P_F$ and $P_R$ represents forward RF electric power and reverse RF electric power.

8. The skin treatment device of claim 7, wherein
the data processor calculates temperature variation inside the skin tissue of the user for respective output levels of the RF energy based on the calculated skin impedance.

9. The skin treatment device of claim 8, wherein

the controller controls the output parameter of the RF energy based on at least one of the calculated skin impedance, the RF output power efficiency, and the temperature variation inside the skin tissue, and
the output parameter of the RF energy is at least one of a voltage size and an output time of the RF energy.

10. The skin treatment device of claim 1, further comprising

a display unit,
wherein at least one of the calculated skin impedance of the user and the RF output power efficiency is output to the display unit.

11. A method for controlling a skin treatment device comprising:

generating RF energy;
measuring a forward RF voltage caused by the RF energy and a reverse RF voltage caused by the RF energy reflected to a RF generator;
calculating skin impedance of a user and RF output power efficiency based on the measured forward RF voltage and the reverse RF voltage; and
controlling an output of the RF generator based on the calculated skin impedance and the RF output power efficiency.

# FIG. 1

FIG. 2

# FIG. 3

COUPLED
302 —○ REVERSE

INTERNAL 50Ω
LOAD

RF INPUT
310 —○

RF OUTPUT
○— 320

INTERNAL 50Ω
LOAD

LOAD

301 —○ COUPLED
FORWARD

FIG. 4

# FIG. 5

| | |
|---|---|
| Output RF energy | S501 |

↓

| | |
|---|---|
| Measure forward RF voltage and reverse RF voltage | S502 |

↓

| | |
|---|---|
| Calculate skin impedance and RF output power efficiency | S503 |

↓

| | |
|---|---|
| Control output parameters of RF energy based on skin impedance and RF output power efficiency | S504 |

**EP 4 667 048 A1**

# INTERNATIONAL SEARCH REPORT

| | International application No. |
|---|---|
| | **PCT/KR2024/001269** |

| | |
|---|---|
| **A.** | **CLASSIFICATION OF SUBJECT MATTER** |
| | **A61N 1/40**(2006.01)i; **A61N 1/32**(2006.01)i; **A61N 1/36**(2006.01)i; **A61N 1/06**(2006.01)i; **A61B 5/0531**(2021.01)i; **A61B 5/01**(2006.01)i; **A61B 5/00**(2006.01)i |
| | According to International Patent Classification (IPC) or to both national classification and IPC |

| | |
|---|---|
| **B.** | **FIELDS SEARCHED** |
| | Minimum documentation searched (classification system followed by classification symbols) |
| | A61N 1/40(2006.01); A61B 5/01(2006.01); A61B 5/053(2006.01); A61B 5/07(2006.01); A61N 1/04(2006.01); A61N 1/06(2006.01); A61N 1/08(2006.01); A61N 1/36(2006.01); A61N 1/372(2006.01); A61N 7/02(2006.01) |
| | Documentation searched other than minimum documentation to the extent that such documents are included in the fields searched |
| | Korean utility models and applications for utility models: IPC as above<br>Japanese utility models and applications for utility models: IPC as above |
| | Electronic data base consulted during the international search (name of data base and, where practicable, search terms used) |
| | eKOMPASS (KIPO internal) & keywords: 고추파(Radio Frequency, RF), 피부(skin), 역방향(reverse direction), 반사 (reflection), 전압(voltage), 전극(electrode), 니들(needle), 디스플레이(display), 아날로그-디지털 컨버터(analog-digital converter, ADC) |

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | KR 10-1725371 B1 (BAE, Dong Ho) 26 April 2017 (2017-04-26)<br>See paragraphs [0031], [0042], [0044], [0066], [0070], [0102], [0104], [0106] and [0120]; claim 1; and figures 1, 3 and 6. | 1-3,10-11 |
| Y | | 4-7 |
| A | | 8-9 |
| Y | US 2022-0152388 A1 (STIMWAVE TECHNOLOGIES INCORPORATED) 19 May 2022 (2022-05-19)<br>See paragraph [0062]; and figures 3A-3B. | 4-7 |

☑ Further documents are listed in the continuation of Box C.   ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **24 May 2024** | **27 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/001269**

**C.    DOCUMENTS CONSIDERED TO BE RELEVANT**

| Category* | Citation of document, with indication, where appropriate, of the relevant passages | Relevant to claim No. |
|---|---|---|
| X | KR 10-1607299 B1 (BAE, Dong Ho) 11 April 2016 (2016-04-11)<br>    See paragraphs [0025]-[0030]; claims 1 and 3-6; and figures 1-5. | 1-3,10-11 |
| Y | | 4-7 |
| A | | 8-9 |
| A | US 2021-0161427 A1 (BOSTON SCIENTIFIC SCIMED, INC.) 03 June 2021 (2021-06-03)<br>    See entire document. | 1-11 |
| A | KR 10-2013-0012040 A (KO, Young San) 31 January 2013 (2013-01-31)<br>    See entire document. | 1-11 |

Form PCT/ISA/210 (second sheet) (July 2022)

**INTERNATIONAL SEARCH REPORT**
Information on patent family members

International application No.

**PCT/KR2024/001269**

| Patent document cited in search report | | | Publication date (day/month/year) | Patent family member(s) | | | Publication date (day/month/year) |
|---|---|---|---|---|---|---|---|
| KR | 10-1725371 | B1 | 26 April 2017 | None | | | |
| US | 2022-0152388 | A1 | 19 May 2022 | AU | 2019-215179 | A1 | 27 August 2020 |
| | | | | CA | 3090274 | A1 | 08 August 2019 |
| | | | | CN | 113348014 | A | 03 September 2021 |
| | | | | EP | 3737465 | A1 | 18 November 2020 |
| | | | | EP | 3737465 | A4 | 13 October 2021 |
| | | | | US | 2019-0232057 | A1 | 01 August 2019 |
| | | | | WO | 2019-152870 | A1 | 08 August 2019 |
| KR | 10-1607299 | B1 | 11 April 2016 | None | | | |
| US | 2021-0161427 | A1 | 03 June 2021 | US | 10925512 | B2 | 23 February 2021 |
| | | | | US | 2017-0258361 | A1 | 14 September 2017 |
| KR | 10-2013-0012040 | A | 31 January 2013 | KR | 10-1300769 | B1 | 29 August 2013 |

Form PCT/ISA/210 (patent family annex) (July 2022)

**REFERENCES CITED IN THE DESCRIPTION**

**Patent documents cited in the description**

- WO 1020180111202 A

- WO 1020220129344 A